# EUROPEAN PATENT APPLICATION

(11) **EP 2 050 405 A2**
(43) Date of publication of application: **22.04.2009**
(21) Application number: 08253356.3
(22) Date of filing: 16.10.2008
(51) Int. Cl.: A61B 17/34, A61B 17/00

(54) **Access port using shape memory anchor**

(30) Priority: 17.10.2007 US 980582 P; 04.08.2008 US 185143
(71) Applicant: Tyco Healthcare Group LP, North Haven, CT 06473 (US)
(72) Inventor: Sung, Jason, North Branford, CT 06471 (US)
(74) Representative: Soames, Candida Jane

(57) **Abstract**

The present disclosure provides an anchoring cannula (10) for use in surgery. The cannula (10) possesses a shape memory polymer (20) on the distal portion thereof which expands upon heating thereby securing the cannula (10) in tissue.

## Description

### CROSS-REFERENCE TO RELATED APPLICATIONS

This application claims the benefit of and priority to U.S. Provisional Patent Application No. 60/980,582, filed October 17, 2007, the entire disclosure of which is incorporated by reference herein.

### TECHNICAL FIELD

The present disclosure provides anchoring cannulas for use in surgical procedures and, more particularly, to an anchoring cannula possessing a shape memory polymer on a distal end thereof which acts to anchor the cannula to tissue upon insertion in a patient's body. Methods of using such a cannula are also provided.

### BACKGROUND

During laparoscopic procedures, cannulas are utilized to provide an access port for surgical instruments and a conduit for introducing insufflation gases into the body cavity. In embodiments, a sharp trocar may be positioned within the cannula and utilized to puncture or pierce the tissue or abdominal wall. Thereafter the trocar may be removed, leaving the cannula in place and insufflation gases forced into the body cavity to form an anatomical operating space.

In order to prevent the cannula from migrating in or out through the incision, some cannulas may be provided with anchoring structures to prevent the cannula from slipping out of the incision. For example, balloons have been used in some devices to assist in anchoring a cannula, as disclosed in U.S. Patent No. 5,468,248 and U.S. Patent Application Publication No. 2004/0138702, the entire disclosures of each of which are incorporated by reference herein. However, unless the anchoring structure is firmly secured against the tissue, leakage of insufflation gases may occur. Thus, means for anchoring cannulas to secure the cannula to the tissue and prevent leakage of insufflation gases remain desirable.

### SUMMARY

The present disclosure provides anchoring cannulas for use in surgical procedures. In embodiments, a cannula of the present disclosure may include an elongated longitudinal shaft having a distal opening, a proximal opening for receipt of operating instruments therethrough, an anchoring member including a shape memory polymer on at least a portion of an outer surface of the elongated longitudinal shaft, and a sliding tube slidably mounted on the outer surface of the cannula.

In embodiments, a cannula of the present disclosure may include an elongated longitudinal shaft having a distal opening, a proximal opening for receipt of operating instruments therethrough, an anchoring member including a shape memory polymer selected from the group consisting of polyurethanes, poly(styrene-butadiene) block copolymers, polynorbomenes, caprolactones, dioxanones, diol esters, ether-ester diols, carbonates, and combinations thereof on at least a portion of an outer surface of the elongated longitudinal shaft, and a sliding tube slidably mounted on the outer surface of the cannula.

In embodiments, the shape memory polymer utilized for the anchoring member may have a temporary shape with a compressed thickness and a permanent shape with an expanded diameter.

In embodiments, medicinal agents may be added to cannulas of the present disclosure.

### BRIEF DESCRIPTION OF THE DRAWINGS

FIG. 1 is a depiction of an anchoring cannula of the present disclosure having a shape memory polymeric anchoring member attached thereto;

FIG. 2 is a depiction of an anchoring cannula of the present disclosure in use having a shape memory polymeric anchoring member expanded to seal the opening of an incision; and

FIGS. 3A and 3B are depictions of another anchoring cannula of the present disclosure having an anchoring member covered by an elastomeric cuff to assist in sealing the opening of an incision.

### DETAILED DESCRIPTION

The present disclosure provides an anchoring cannula including a cannula body having a hollow shaft, and an anchoring member affixed to the distal end of the shaft. The anchoring member may be made of a shape memory polymer which expands upon application of heat, such as body heat, to secure the distal end of the cannula within the opening of an incision in the body.

There is also disclosed a method of securing a cannula to tissue which includes providing the disclosed cannula. The cannula is inserted through an incision in the tissue to position the expandable member at the opening of the incision and the expandable member is thereafter expanded by the application of heat, such as body heat. As noted above, the expandable member may be made of a shape memory polymer.

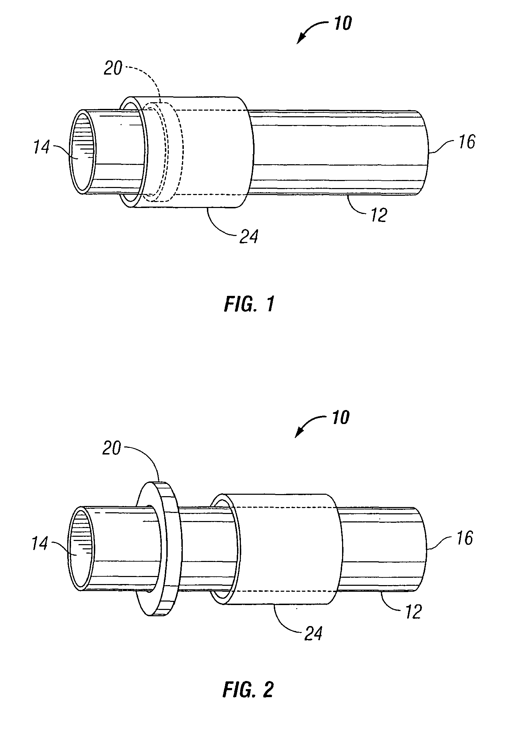

As depicted in FIG. 1, anchoring cannula 10 generally includes an elongated cannula shaft 12. Cannula 10 and shaft 12 may be formed of any material. Shaft 12 has a distal opening 14 and a proximal opening 16 for receipt of operating instruments therethrough. An anchoring member made of a shape memory polymer 20 is provided on a distal end of shaft 12. Cannula 10 may also include an insufflation port (not shown) which is configured to receive a syringe containing insufflation fluid and is in fluid communication with the interior of shaft 12 so as to provide insufflation fluid into the body. Shape-polymeric anchoring member 20 may be secured to shaft 12 in any way, including the use of chemical and physical elements such as adhesives, sealants, glues, and the like or, in some embodiments, the use of locking rings (not shown) located on the proximal and distal ends, respectively, of shape-polymeric anchoring member 20. In other embodiments, the anchoring member may be compressed to a dimension fitting within a recess or shallow depression on the surface of the distal end of cannula 10.

In embodiments, as depicted in FIG. 1, the anchoring cannula 10 of the present disclosure may also have an optional sliding tube 24 slidably mounted on an outer surface of cannula 10. The sliding tube 24 acts as a cover of the shape-memory polymeric anchoring member 20 to prevent premature expansion of same. As described in greater detail below, sliding tube 24 may also be utilized to facilitate removal of the cannula 10 after use.

As noted above, the anchoring member 20 of cannula 10 may be formed of shape memory polymeric materials. Such materials possess a permanent shape and a temporary shape. In embodiments, the temporary shape is of a configuration which enhances the ability of one to introduce a cannula possessing an anchoring member into a patient's body, while the permanent shape is of a configuration which enhances the retention of the cannula at the site of an incision. Such materials include, for example, polyurethanes, poly(styrene-butadiene) block copolymers, polynorbomenes, caprolactones, dioxanones, diol esters including oligo (epsilon caprolactone) diol, lactic acid, lactide, glycolic acid, glycolide, ether-ester diols including oligo (p-dioxanone) diol, carbonates including trimethylene carbonate, combinations thereof, and the like. In embodiments, the shape memory polymer may be a copolymer of two components with different thermal characteristics, such as oligo (epsilon-caprolactone) dimethacrylates and butyl acrylates including poly(epsilon-caprolactone) dimethacrylate-poly (n-butyl acrylate), or a diol ester and an ether-ester diol such as oligo (epsilon caprolactone) diol/oligo (p-dioxanone) diol copolymers. These multi-block oligo (epsilon-caprolactone) diol/oligo (p-dioxanone) diol copolymers possess two block segments: a "hard" segment and a "switching" segment linked together in linear chains. Such materials are disclosed, for example, in Lendlein, "Shape Memory Polymers-Biodegradable Sutures," Materials World, Vol. 10, no. 7, pp. 29-30 (July 2002), the entire disclosure of which is incorporated by reference herein. In other embodiments, blends of bioabsorbable materials may be utilized including, but not limited to, urethanes blended with lactic acid and/or glycolic acid, homopolymers thereof or copolymers thereof, and acrylates blended with caprolactones such as polycaprolactone dimethacrylate poly(butyl acrylate) blends, and combinations thereof.

Other examples of these shape memory polymers and methods for forming permanent and temporary shapes therewith are set forth in Lendlein et al., "Shape memory polymers as stimuli-sensitive implant materials," Clinical Hemorheology and Microcirculation, 32 (2005) 105-116, and Lendlein et al., "Biodegradable, Elastic Shape-Memory Polymers for Potential Biomedical Applications," Science, Vol. 269 (2002) 1673-1676, the entire disclosures of each of which are incorporated by reference herein.

The shape memory polymeric materials of the present disclosure may, in embodiments, be compressed or expanded into temporary forms up to about four times larger or four times smaller than their permanent shape. Thus, in embodiments, shape memory polymeric materials may be fashioned into an anchoring member having a permanent shape that possesses an expanded diameter up to about four times greater than its compressed temporary diameter.

In embodiments, a molding process may be utilized to produce the anchoring member of the present disclosure. Plastic molding methods may be employed and include, but are not limited to, melt molding, solution molding and the like. Injection molding, extrusion molding, compression molding and other methods can also be used as the melt molding technique. Once placed in the mold with the proper dimensions, the anchoring member may be heated to a suitable temperature, in embodiments at a temperature referred to as the permanent temperature (Tₚₑᵣₘ) which may, in embodiments, be the melting temperature of the shape memory polymeric material utilized to form the anchoring member. Heating of the anchoring member may be at suitable temperatures including, for example, from about 40° C to about 180° C, in embodiments from about 80° C to about 150° C, for a period of time from about 10 minutes to about 60 minutes, in embodiments from about 15 minutes to about 20 minutes, to obtain the permanent shape and dimensions, including thickness.

After the anchoring member with the desired thickness has been formed, the anchoring member may be deformed at a deforming temperature to obtain, in embodiments, a thinner temporary shape. Suitable temperatures for deformation will vary depending on the shape memory polymer utilized, but generally may be above the transition temperature of the polymer (Tₜᵣₐₙₛ), but below the Tₚₑᵣₘ. In embodiments, the shape memory polymer may be cooled from its Tₚₑᵣₘ to a lower temperature which remains above the Tₜᵣₐₙₛ and deformed, in embodiments by compression, to a thinner temporary shape. In other embodiments, the anchoring member may be compressed and cooled to room temperature (about 20° C to about 25° C) to obtain its thinner temporary straight shape, although the temperature may differ depending upon the particular polymer employed. The anchoring member may then be cooled to a temperature below Tₜᵣₐₙₛ, at which time the anchoring member of the present disclosure may be affixed to the anchoring cannula as described above and is ready for use. As the Tₜᵣₐₙₛ is usually greater than room temperature, in embodiments cooling to room temperature may be sufficient to form the temporary shape.

The temperature for deformation treatment of the anchoring member molded with a previously memorized thick shape is one that makes possible ready deformation without producing cracks and should not exceed the temperature adopted for the shape memorization (e.g., Tₚₑᵣₘ)_{.} Deformation treatment at a temperature exceeding that for the original shape memorization may cause the object to memorize a new deformed shape.

There are no particular limitations on the manner in which the deformation can be achieved. Deformation can be achieved either by hand or by way of a suitable device selected to provide the desired thickness to the anchoring member.

The anchoring member may be deformed to its temporary shape prior to its attachment to the cannula or, in other embodiments, the anchoring member may be deformed to its temporary shape after attachment to the cannula.

In order to keep the shape and thickness of the anchoring member in its temporary shape, the shape-memory anchoring member of the present disclosure should be stored at a temperature which will not cause plastic deformation of the polymers or premature switching to their permanent shape. In embodiments, the shape-memory anchoring member may be stored in a refrigerator.

The anchoring member thus prepared recover their originally memorized thicker shape on heating, either by placement in a patient's body or the addition of exogenous heat at a prescribed temperature, in embodiments above Tₜᵣₐₙₛ for the shape memory polymer utilized. In embodiments, the shape polymeric material utilized to anchor a cannula in an incision may have a temporary shape possessing a thickness, referred to in embodiments as a compressed annular thickness, from about 0.02 inches to about 0.1 inches, in embodiments from about 0.05 inches to about 0.08 inches, and expand to an annular diameter from about 0.75 inches to about 1.75 inches, in embodiments from about 1 inch to about 1.5 inches, referred to herein as its permanent shape or its expanded diameter, to anchor a cannula at the opening of an incision.

As the anchoring member of the present disclosure are utilized in a living body, heating with body heat (about 37° C) is possible. In such a case, the temperature for shape memorization should be as low as possible and the recovery of the memorized shape may occur fairly slowly.

However, in some embodiments a higher shape-memory temperature may be desirable in order to make the shape recover at a slightly higher temperature than body temperature. Thus, in some cases releasing the anchoring member from deformation to recover the originally memorized thicker shape can be achieved by heating. On heating at a temperature from about 30° C to about 50° C, in embodiments from about 39° C to about 43° C, the temporary thin shape may be released and the memorized permanent thick shape recovered. The higher the temperature for heating, the shorter the time for recovery of the originally memorized shape. The methods for this heating are not limited. Heating can be accomplished by using a gas or liquid heating medium, heating devices, ultrasonic waves, or the like. Of course, in an application involving a living body, care may be taken to utilize a heating temperature which will not cause burns. When a liquid heating medium is used, physiological saline solution or alcohol may be desirable.

Similarly, in other embodiments electrically active polymers, also known as electroactive polymers, which can alter their configuration upon application of electricity, may be utilized to fashion anchoring member to secure a cannula within the body. Suitable examples of electroactive polymers include poly(aniline), substituted poly(aniline)s, polycarbazoles, substituted polycarbazoles, polyindoles, poly(pyrrole)s, substituted poly(pyrrole)s, poly(thiophene)s, substituted poly(thiophene)s, poly(acetylene)s, poly(ethylene dioxythiophene)s, poly(ethylenedioxypyrrole)s, poly(p-phenylene vinylene)s, and the like, or combinations including at least one of the foregoing electroactive polymers. Blends or copolymers or composites of the foregoing electroactive polymers may also be used.

Similar to the change in shape which a shape memory material may undergo upon the application of energy, such as heat, in embodiments an electroactive polymer may undergo a change in shape upon the application of electricity from a low voltage electrical source (such as a battery). Electricity may also be utilized, in embodiments, to promote the change in shape of a shape memory alloy such as Nitinol. Suitable amounts of electricity which may be applied to effect such change will vary with the electroactive polymer or shape memory alloy utilized, but can be from about 5 volts to about 30 volts, in embodiments from about 10 volts to about 20 volts. The application will result in the anchoring member constructed of the electroactive polymer to change its shape to an anchoring structure capable of anchoring a cannula at the site of an incision.

While an electroactive polymer does not have the same permanent shape and temporary shape as those terms are described above with respect to shape memory polymers, as used herein the term "permanent shape" as applied to an electroactive polymer may refer to, in embodiments, the shape the electroactive polymer adopts upon the application of electricity, and the term "temporary shape" as applied to an electroactive polymer may refer to, in embodiments, the shape the electroactive polymer adopts in the absence of electricity.

Referring to FIGS. 1 and 2, the use of anchoring cannula 10 will now be described. In this embodiment, the anchoring member 20 remains at the site of incision and expands to seal the tissue at the opening of the incision itself. As depicted in FIG. 1, in embodiments cannula 10 may possess anchoring member 20 and sliding tube 24 located over an outer surface of cannula 10. As depicted in FIG. 1, before placement at the site of an incision, the anchoring member 20 has been compressed into its temporary shape, e.g., its thinner, compressed diameter and may, in embodiments, be covered by sliding tube 24. As noted above, sliding tube 24 slidably mounted on outer surface of cannula 10 may thus be utilized to assist the anchoring member in retaining its thinner temporary shape.

A sharp tip trocar (not shown) may be inserted through distal opening 14 and is used to puncture the abdominal wall. Any other instrument suitable for penetrating the abdominal wall may be used instead of a sharp tip trocar, including bladeless trocars, and the like. As depicted in FIG. 2, sliding tube 24 may be moved proximally to expose anchoring member 20 just prior to the insertion of cannula 10 at the site of an incision. Upon placement of anchoring member 20 at the opening of an incision, the heat of the patient or the application of heat from an exogenous source may be used to heat anchoring member 20 whereby it recovers its permanent shape, e.g., its thicker, expanded diameter as depicted in FIG. 2, so that it fills and thereby seals the opening of incision and anchors cannula 10 by contacting the tissue surrounding the opening of the incision. In embodiments, the heat of the body (about 37° C), may be sufficient for the anchoring member to return to its thicker, permanent shape. In other embodiments, heat may be applied to the anchoring member, in embodiments from about 39° C to about 43° C (just above human body temperature), to enhance the return of the shape memory polymer to its permanent shape.

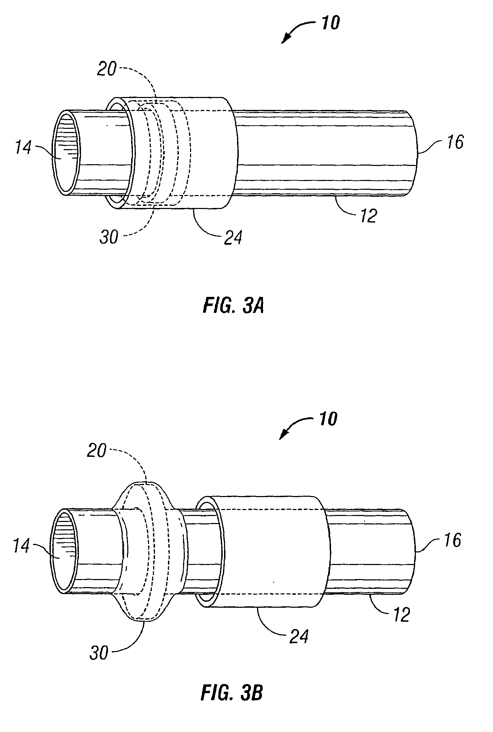

As depicted in FIGS. 3A and 3B, in some embodiments cannula 10 of the present disclosure having elongated longitudinal shaft 12, distal opening 14, proximal opening 16, anchoring member 20, and sliding tube 24, may also possess an elastomeric cuff 30 over the outer surface of anchoring member 20. As the anchoring member 20 expands to its permanent shape, the elastomeric cuff 30 expands to further aid in the complete sealing of the incision as depicted in FIG. 3B. Any biocompatible elastomeric material may be utilized to fashion elastomeric cuff 30. In embodiments, suitable elastomeric materials include polyurethanes, polytetrafluoroethylenes, silicone rubbers, thermoplastic elastomers, and the like.

Once cannula 10 has been secured within the opening of an incision, an insufflation fluid or gas may by forced through distal opening 14 and into the body cavity and surgical instruments and similar devices may be introduced through cannula 10 during a laparoscopic procedure. After an operation has been performed, sliding tube 24 may be slid distally to compress the anchoring member 20 to its original temporary thin, compressed diameter shape (as depicted in FIG. 1) thereby enclosing the anchoring member inside the sliding tube, as well as any elastomeric cuff 30, if present (as depicted in FIG. 3A), facilitating withdrawal of the cannula from the body.

In embodiments, it may be desirable to add additional components, including medicinal agents, to the shape memory polymers or elastomers utilized to form elastomeric cuffs of the anchoring member of the present disclosure. The term "medicinal agent", as used herein, is used in its broadest sense and includes any substance or mixture of substances that have clinical use. Consequently, medicinal agents may or may not have pharmacological activity per se, e.g., a dye. Alternatively, a medicinal agent could be any agent which provides a therapeutic or prophylactic effect, a compound that affects or participates in tissue growth, cell growth, cell differentiation, a compound that may be able to invoke a biological action such as an immune response, or could play any other role in one or more biological processes.

Examples of classes of medicinal agents which may be utilized in accordance with the present disclosure include antimicrobials; analgesics; anesthetics; antiinflammatory agents such as hormonal agents, hydrocortisone, prednisolone, prednisone, non-hormonal agents, allopurinol, indomethacin, phenylbutazone and the like; diagnostic agents; hemostats to halt or prevent bleeding; anticoagulants; antibiotics; anti-fungals; anti-virals; and immunological agents.

Suitable antimicrobial agents which may be included as a medicinal agent with the shape memory polymers utilized to form the anchoring member of the present disclosure include triclosan, also known as 2,4,4'-trichloro-2'-hydroxydiphenyl ether, chlorhexidine and its salts, including chlorhexidine acetate, chlorhexidine gluconate, chlorhexidine hydrochloride, and chlorhexidine sulfate, silver and its salts, including silver acetate, silver benzoate, silver carbonate, silver citrate, silver iodate, silver iodide, silver lactate, silver laurate, silver nitrate, silver oxide, silver palmitate, silver protein, and silver sulfadiazine, polymyxin, tetracycline, aminoglycosides, such as tobramycin and gentamicin, rifampicin, bacitracin, neomycin, chloramphenicol, miconazole, quinolones such as oxolinic acid, norfloxacin, nalidixic acid, pefloxacin, enoxacin and ciprofloxacin, penicillins such as oxacillin and pipracil, nonoxynol 9, fusidic acid, cephalosporins, and combinations thereof. In addition, antimicrobial proteins and peptides such as bovine lactoferrin and lactoferricin B may be included as a medicinal agent with the shape memory polymers utilized to form the anchoring member of the present disclosure.

Examples of hemostat materials which can be employed include fibrin-based, collagen-based oxidized regenerated cellulose-based, and gelatin-based topical hemostats. Examples of commercially available hemostat materials include fibrinogen-thrombin combination materials sold under the trade designations CoStasis^{™} by Tyco Healthcare Group, LP, and Tisseel^{™} sold by Baxter International, Inc. Hemostats herein also include astringents, for example, aluminum sulfate, and coagulants.

A single medicinal agent may be utilized with the shape memory polymers or elastomers utilized to form the elastomeric cuffs of the anchoring member of a cannula of the present disclosure or, in alternate embodiments, any combination of medicinal agents may be utilized.

The medicinal agent may be disposed on a surface of the anchoring member, elastomeric cuff, or both, or impregnated in or combined with the shape memory polymers or elastomers utilized to form the elastomeric cuffs of the anchoring member of a cannula of the present disclosure.

Cannulas of the present disclosure possessing anchoring member made of shape memory polymeric materials may, in embodiments, avoid the need for extra deploying action, including that required with an anchoring balloon. The materials utilized to make the anchoring member are durable, the design is simple, and the cannula with the anchoring member of the present disclosure is simple to make utilizing existing materials and manufacturing processes. Thus, the present disclosure provides a cannula which is both economical and easy to use.

While the above description contains many specifics, these specifics should not be construed as limitations on the scope of the disclosure, but merely as exemplifications of embodiments thereof. Those skilled in the art will envision many other possibilities within the scope and spirit of the disclosure as defined by the claims appended hereto.
The present disclosure provides an anchoring cannula for use in surgery. The cannula possesses a shape memory polymer on the distal portion thereof which expands upon heating thereby securing the cannula in tissue.

## Claims

1. A cannula comprising:
an elongated longitudinal shaft having a distal opening, a proximal opening for receipt of operating instruments therethrough, an anchoring member comprising a shape memory polymer on at least a portion of an outer surface of the elongated longitudinal shaft, and a sliding tube slidably mounted on the outer surface of the cannula.

2. The cannula of claim 1, wherein the shape memory polymer is selected from the group consisting of polyurethanes, poly(styrene-butadiene) block copolymers, polynorbomenes, caprolactones, dioxanones, diol esters, ether-ester diols, carbonates, and combinations thereof.

3. The cannula of claim 1, wherein the shape memory polymer is selected from the group consisting of oligo (epsilon caprolactone) diol, lactic acid, lactide, glycolic acid, glycolide, oligo (p-dioxanone) diol, trimethylene carbonate, and combinations thereof.

4. The cannula of claim 1, wherein the shape memory polymer is selected from the group consisting of poly(styrene-butadiene) copolymers, oligo (epsilon caprolactone) diol/oligo (p-dioxanone) diol copolymers, and poly(epsilon-caprolactone) dimethacrylate-poly (n-butyl acrylate) copolymers.

5. The cannula of claim 1, wherein the shape memory polymer comprises a blend of materials selected from the group consisting of urethanes, lactic acid, glycolic acid, acrylates, caprolactones, homopolymers thereof, copolymers thereof, and combinations thereof.

6. The cannula of claim 1, wherein the shape memory polymer undergoes a change in shape at a temperature from about 30° C to about 50° C.

7. The cannula of 1, wherein the anchoring member is secured to the shaft by at least one of chemical methods and physical methods.

8. The cannula of claim 7, wherein the anchoring member is secured to the shaft chemically by at least one of adhesives, sealants, and glues.

9. The cannula of claim 7, wherein the anchoring member is secured to the shaft physically by locking rings located on the proximal end and distal end of the anchoring member.

10. The cannula of claim 1, wherein the sliding tube covers the shape-memory polymeric anchoring member prior to use.

11. The cannula of claim 1, wherein the anchoring member has a permanent shape comprising an expanded diameter of from about 0.75 inches to about 1.75 inches and a temporary shape comprising a compressed thickness of from about 0.02 inches to about 0.1 inches.

12. The cannula of claim 1, wherein the anchoring member further comprises a medicinal agent.

13. A cannula comprising:
an elongated longitudinal shaft having a distal opening, a proximal opening for receipt of operating instruments therethrough, an anchoring member comprising a shape memory polymer selected from the group consisting of polyurethanes, poly(styrene-butadiene) block copolymers, polynorbomenes, caprolactones, dioxanones, diol esters, ether-ester diols, carbonates, and combinations thereof on at least a portion of an outer surface of the elongated longitudinal shaft, and a sliding tube slidably mounted on the outer surface of the cannula.

14. The cannula of claim 13, wherein the shape memory polymer is selected from the group consisting of oligo (epsilon caprolactone) diol, lactic acid, lactide, glycolic acid, glycolide, oligo (p-dioxanone) diol, trimethylene carbonate, and combinations thereof.

15. The cannula of claim 13, wherein the shape memory polymer is selected from the group consisting of poly(styrene-butadiene) copolymers, oligo (epsilon caprolactone) diol/oligo (p-dioxanone) diol copolymers, and poly(epsilon-caprolactone) dimethacrylate-poly (n-butyl acrylate) copolymers.

16. The cannula of claim 13, wherein the shape memory polymer comprises a blend of materials selected from the group consisting of urethanes, lactic acid, glycolic acid, acrylates, caprolactones, homopolymers thereof, copolymers thereof, and combinations thereof.

17. The cannula of claim 13, wherein the anchoring segment comprises a shape memory polymer which undergoes a change in shape at a temperature from about 30° C to about 50° C.

18. The cannula of claim 13, wherein the sliding tube covers the shape-memory polymeric anchoring member prior to use.

19. The cannula of claim 13, wherein the anchoring member has a permanent shape comprising an expanded diameter of from about 0.75 inches to about 1.75 inches and a temporary shape comprising a compressed thickness of from about 0.02 inches to about 0.1 inches.

20. The cannula of claim 13, wherein the anchoring member further comprises a medicinal agent.
